# EUROPEAN PATENT APPLICATION

(11) **EP 3 199 129 A1**
(43) Date of publication of application: **02.08.2017**
(21) Application number: 16170972.0
(22) Date of filing: 24.05.2016
(51) Int. Cl.: A61F 5/02

(54) **SPINE SUPPORT AND TRACTION APPARATUS**

(30) Priority: 26.01.2016 KR 20160009234
(71) Applicant: Kang, Min Young, Seoul 04969 (KR)
(72) Inventor: Kang, Min Young, Seoul 04969 (KR)
(74) Representative: Grootscholten, Johannes A.M.

(57) **Abstract**

Provided is a spine support and traction apparatus including a support frame extended in a length direction of a spine of a user and a compression belt connected to the support frame and configured to apply an external force to the support frame, and wherein the compression belt may include an expansion zone and the expansion zone may be provided to generate traction force for the spine by expansion of the expansion zone.

## Description

### CROSS-REFERENCE TO RELATED APPLICATION

This application claims the priority benefit of Korean Patent Application No. 10-2016-0009234, filed on January 26, 2016, in the Korean Intellectual Property Office, the disclosure of which is incorporated herein by reference.

### BACKGROUND

### 1. Field

One or more example embodiments relate to a spine support and traction apparatus, and more particularly, to a spine support and traction apparatus that includes a portion of a support frame configured to cover a pelvis/lumbar vertebra and another portion of the support frame configured to cover a bacls/thoracic vertebra to support a spine, and that may effectively generate a tractive force for the spine by applying an external force to the support frame by expansion of a compression belt.

### 2. Description of Related Art

In general, a spine is a central human body skeleton and an essential human structure that supports a human body, and acts as a column of a human body to which ligaments, muscles, cartilages, and the like are complexly interconnected.

The spine includes various joints and nerves, for example, a vertebral body that acts as a column of a human body and an intervertebral disc that enables joint movements. The spine also plays a role as an axis that maintains a balance in both a static state and a motion state, as a column that supports a weight of an upper body by various structures being collected, and as a joint that enables an upper extremity and a lower extremity to conduct various tasks through bending and stretching movements.

In addition, the spine is extended upwards and downwards to be connected to a cervical vertebra that supports a head portion of a human body. The spine and the cervical vertebra include various joints, and discs that buffer shocks or impacts are disposed between the joints. The spine and the cervical vertebra also include various neural lines that receive a signal from a cranial nerve and transfer the received signal to each muscle and sensory portion.

Thus, the spine plays a significant role in activities performed by a human body.

However, when the spine acting as a column that is considered most important in a human body stays in a wrong posture for a long period of time, or when the cervical vertebra in a head side and a lumbar vertebra in a pelvic side are twisted due to a severe impact from an external cause such as, for example, a traffic accident, and thereby pressing against adjacent nerves, spinal diseases such as, for example, scoliosis, lumber disc disease, lumbago, and dyspepsia may be developed.

Therefore, various spinal orthoses and supports have been developed to protect a spine that plays a pivotal role in a human body skeleton.

For example, Korean Patent Application No. 10-2013-0103943 filed on August 30, 2013, discloses a seat mounted apparatus for spine traction using a weight of a user.

### SUMMARY

An aspect provides a spine support and traction apparatus that may include a portion of a support frame configured to cover a pelvis/lumbar vertebra and another portion of the support frame configured to cover a back/thoracic vertebra to support a spine, and that may effectively generate a tractive force for the spine by applying an external force to the support frame by expansion of a compression belt.

The compression belt may allow the support frame to be more closely attached to the spine, and thus may prevent the support frame from being excessively separate from the spine.

The compression belt may include a blocking member to form a flow path through which a fluid moves and facilitate bending of the compression belt.

The compression belt and the support frame may be attachable and detachable, and thus replacing the compression belt and the support frame may be readily performed. In addition, the support frame may be disassembled, and thus replacing and newly assembling the support frame to be suitable for a body type of a user may be readily performed.

The spine support and traction apparatus may be conveniently wearable for anyone irrespective of time and place, and thus may alleviate a muscular pain or help treat spinal diseases in real life.

According to an aspect, there is provided a spine support and traction apparatus including a support frame extended in a length direction of a spine, and a compression belt connected to the support frame and configured to apply an external force to the support frame. The compression belt may include an expansion zone, and the expansion zone may be provided to generate traction force for the spine by expansion of the expansion zone.

The support frame may include a first frame configured to cover a pelvis, a second frame disposed separately from the first frame in the length direction of the spine and configured to cover a torso, and a third frame extended in the length direction of the spine to connect the first frame and the second frame.

The expansion zone may be provided as a plurality of expansion zones, and the plurality of expansion zones may include a first expansion zone disposed in the third frame and configured to apply an external force to the third frame, a second expansion zone connected to one end of the first expansion zone and configured to cover one side of the torso, and a third expansion zone connected to another end of the first expansion zone and configured to cover another side of the torso. The second expansion zone or the third expansion zone may include a fluid inlet into which a fluid is injected to contract or expand the plurality of expansion zones.

The compression belt may further include an attachable and detachable portion configured to attach and detach the compression belt to and from the third frame. The attachable and detachable portion may be disposed at a center of the first expansion zone.

Inflow of the fluid into the attachable and detachable portion may be blocked, and the fluid flowing in the first expansion zone may make a detour around the attachable and detachable portion.

The second expansion zone and the third expansion zone each may include a plurality of blocking members to block inflow of the fluid, and the fluid may be conveyed through a space between neighboring blocking members among the plurality of blocking members.

The plurality of blocking members may be extended in the length direction of the spine in the second expansion zone and the third expansion zone, and may be disposed separately from one another in a lateral direction against the length direction of the spine.

Some blocking members among the plurality of blocking members may be disposed at both ends of the first expansion zone, and a separation distance between blocking members disposed at one end of the first expansion zone and blocking members disposed at the other end of the first expansion zone may correspond to a width of the third frame.

The first expansion zone may be contracted or expanded by the fluid injected into the fluid inlet. When the first expansion zone is contracted, a length of the first expansion zone may be less than a separation distance between the first frame and the second frame. When the first expansion zone is expanded, the length of the first expansion zone may correspond to the separation distance between the first frame and the second frame.

Additional aspects of example embodiments will be set forth in part in the description which follows and, in part, will be apparent from the description, or may be learned by practice of the disclosure.

### BRIEF DESCRIPTION OF THE DRAWINGS

These and/or other aspects, features, and advantages of the invention will become apparent and more readily appreciated from the following description of example embodiments, taken in conjunction with the accompanying drawings of which:
FIGS. 1A and 1B are perspective views of a spine support and traction apparatus according to an example embodiment;
FIG. 2 is a rear view of a support frame according to an example embodiment;
FIG. 3 is an exploded perspective view of a support frame according to an example embodiment;
FIGS. 4A through 4C are views of a compression belt when being contracted according to an example embodiment; and
FIGS. 5A through 5C are views of a compression belt when being expanded according to an example embodiment.

### DETAILED DESCRIPTION

Hereinafter, some example embodiments will be described in detail with reference to the accompanying drawings. Regarding the reference numerals assigned to the elements in the drawings, it should be noted that the same elements will be designated by the same reference numerals, wherever possible, even though they are shown in different drawings. Also, in the description of embodiments, detailed description of well-known related structures or functions will be omitted when it is deemed that such description will cause ambiguous interpretation of the present disclosure.

The terminology used herein is for the purpose of describing particular embodiments only and is not intended to be limiting. As used herein, the singular forms "a," "an," and "the," are intended to include the plural forms as well, unless the context clearly indicates otherwise. In addition, terms such as first, second, A, B, (a), (b), and the like may be used herein to describe components. Each of these terminologies is not used to define an essence, order or sequence of a corresponding component but used merely to distinguish the corresponding component from other component(s). It should be noted that if it is described in the disclosure that one component is "connected," "coupled," or "joined" to another component, a third component may be "connected," "coupled," and "joined" between the first and second components, although the first component may be directly connected, coupled or joined to the second component.

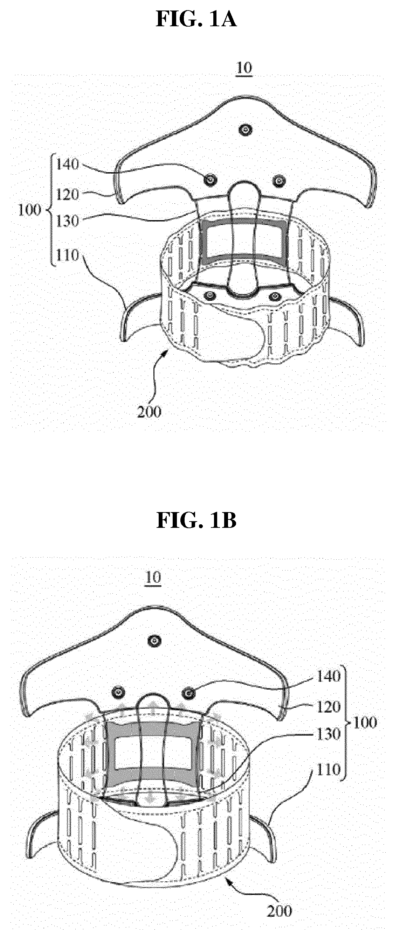

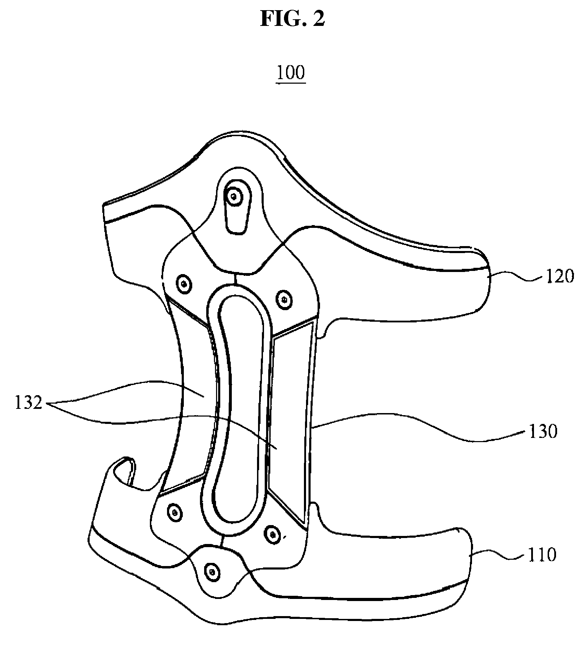

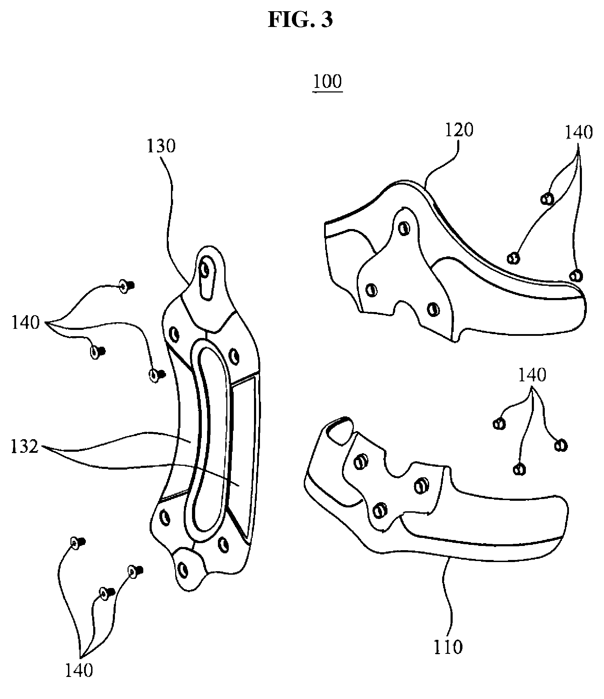

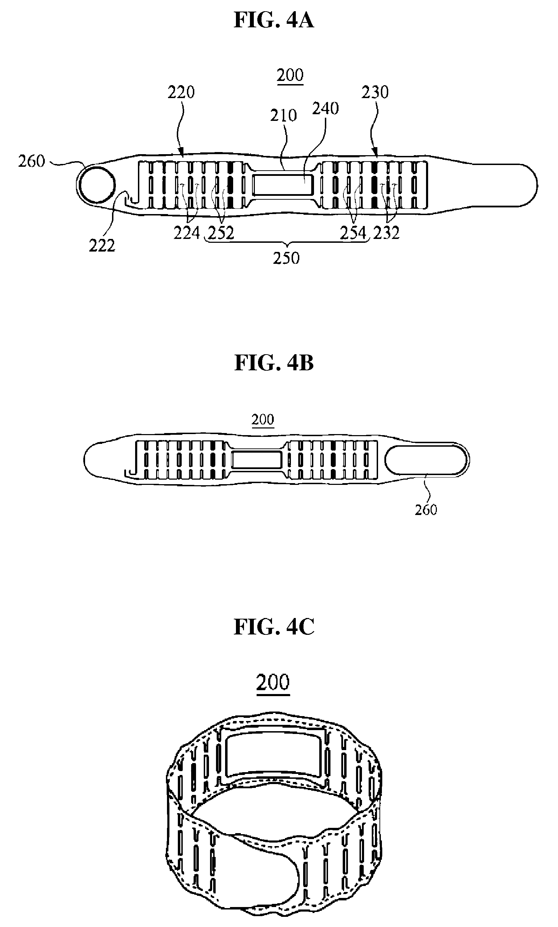

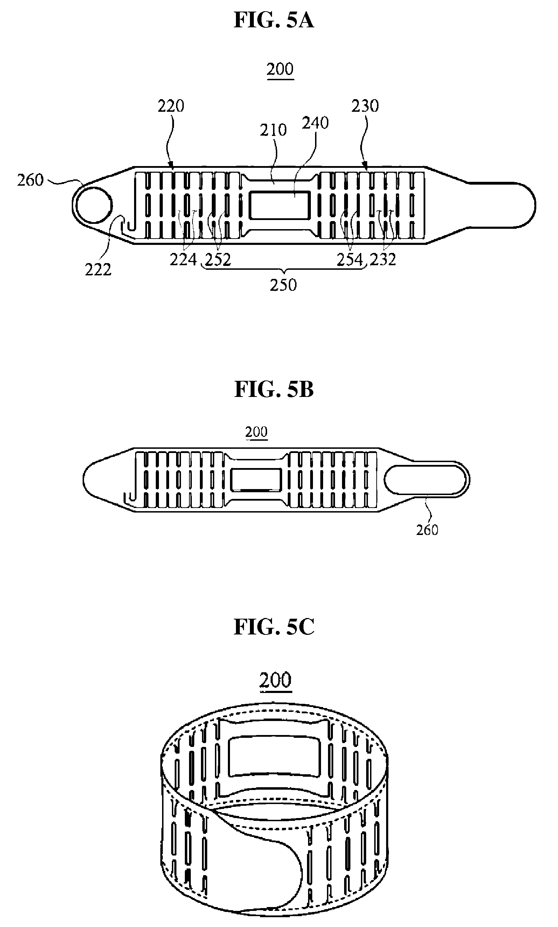

FIGS. 1A and 1B are perspective views of a spine support and traction apparatus according to an example embodiment. FIG. 2 is a rear view of a support frame according to an example embodiment. FIG. 3 is an exploded perspective view of a support frame according to an example embodiment. FIGS. 4A through 4C are views of a compression belt when being contracted according to an example embodiment. FIGS. 5A through 5C are views of a compression belt when being expanded according to an example embodiment.

Referring to FIGS. 1A and 1B, a spine support and traction apparatus 10 includes a support frame 100 and a compression belt 200.

The spine support and traction apparatus 10 may be worn on a spine of a user. The support frame 100 may be extended in a length direction of the spine, and the compression belt 200 may be connected to the support frame 100 to apply an external force to the support frame 100.

For example, when the spine support and traction apparatus 10 is being worn on the spine of the user, the support frame 100 may be disposed on a back of the user and the compression belt 200 may be provided to cover an outer side of the support frame 100.

Thus, similarly to a thoracolumbosacral orthosis (TLSO), the support frame 100 may support a thoracolumbosacral portion, and the compression belt 200 may provide traction or a tractive force for a lumbar vertebra.

Referring further to FIGS. 2 and 3, the support frame 100 includes a first frame 110, a second frame 120, and a third frame 130.

The first frame 110 may be formed of, for example, a stiff material, to cover the pelvis and the lumbar vertebra. Thus, a central portion of the first frame 110 may be formed to be flat in order to be stably put on the spine, and both ends of the first frame 110 may be formed to be curved in order to cover the pelvis or the lumbar vertebra.

The second frame 120 may be disposed upwards in the length direction of the spine to be separate from the first frame 110. Thus, the second frame 120 may be formed to cover the back or a torso of the user. A central portion of the second frame 120 may be formed to be flat in order to be stably put on the spine, and both ends of the second frame 120 may be formed to be curved in order to cover the back or the torso.

The third frame 130 may be extended between the first frame 110 and the second frame 120 in the length direction of the spine, and both ends of the third frame 130 may be connected to the first frame 110 and the second frame 120, respectively.

The third frame 130 may be formed to be curved to correspond to a shape of the spine. A central portion of the third frame 130 may include an empty space extended in the length direction of the spine.

The support frame 100 may further include a fastening member 140 to fasten and disassemble the first frame 110, the second frame 120, and the third frame 130.

FIGS. 2 and 3 illustrate a portion of the support frame 100 that is opposite to a portion disposed on the spine. The fastening member 140, which is a plurality of fastening members, may be disposed between the first frame 110 and the third frame 130. In addition, the fastening member 140, which is a plurality of fastening members, may be disposed between the second frame 120 and the third frame 130.

A back face of the first frame 110 may include a groove to be fastened to one end of the third frame 130. A back face of the second frame 120 may include a groove to be fastened to another end of the third frame 130. Thus, while the third frame 130 is being fastened to the first frame 110 and the second frame 120, an uneven portion may not be formed on a back face of the support frame 100.

In addition, in a structure in which fastening or disassembling the first frame 110, the second frame 120, and the third frame 130 is possible, the first frame 110, the second frame 120, and the third frame 130 may be replaced and newly assembled based on the body type of the user.

For example, when a size of the torso of the user is relatively large or small, or when a length of the spine of the user is relatively long, suitable sizes of the first frame 110, the second frame 120, and the third frame 130 may be selected and newly assembled.

Thus, the spine support and traction apparatus 10 may be customized, and thus may generate a desired or suitable spine tractive force based on a user.

A back face of the third frame 130 may include an attachable and detachable member 132 to be attached to and detached from the compression belt 200.

The attachable and detachable member 132 may be provided as, for example, a Velcro, and disposed on the back face of the third frame 130 to be extended in a length direction of the third frame 130 or in the length direction of the spine.

The compression belt 200 may also include an attachable and detachable member corresponding to the attachable and detachable member 132. A detailed description of such an attachable and detachable member will be provided hereinafter.

Although the attachable and detachable member 132 is provided as Velcro as an example, the attachable and detachable member 132 is not limited thereto and any form that may effectively attach and detach the third frame 130 and the compression belt 200 may be used.

A detailed description of the support frame 100 is provided above, and a detailed description of the compression belt 200 will be provided hereinafter.

Referring to FIGS. 4A through 4C, and 5A through 5C, the compression belt 200 includes a plurality of expansion zones, and traction may be provided for the spine due to expansion of the plurality of expansion zones.

The plurality of expansion zones includes a first expansion zone 210, a second expansion zone 220, and a third expansion zone 230.

The first expansion zone 210 may be disposed in the third frame 130. The second expansion zone 220 may be connected to one end of the first expansion zone 210 to cover one side of the torso, and the third expansion zone 230 may be connected to another end of the first expansion zone 210 to cover another side of the torso.

The second expansion zone 220 or the third expansion zone 230 may include a fluid inlet 222 into which a fluid is injected to contract or expand the plurality of expansion zones.

A gas such as air or a fluid such as water may be injected into the fluid inlet 222, and any form may be possible to be injected to expand or contract the plurality of expansion zones.

Hereinafter, an example of the fluid inlet 222 provided in the second expansion zone 220 will be described. Here, air flows in the fluid inlet 222.

The fluid inlet 222 is provided at one end of the second expansion zone 220, and the air flowing in through the fluid inlet 222 may be conveyed to the third expansion zone 230 through the first expansion zone 210 after passing through the second expansion zone 220.

Here, a flow path may be formed in the compression belt 200, for example, formed among the first expansion zone 210, the second expansion zone 220, and the third expansion zone 230, to convey a fluid in one direction, and thus an additional fluid inlet may not need to be provided in the third expansion zone 230.

In addition, the second expansion zone 220 and the third expansion zone 230 may include a plurality of blocking members 250.

For example, the plurality of blocking members 250 may include a first blocking member 252 provided in the second expansion zone 220 and a second blocking member 254 provided in the third expansion zone 230.

The first blocking member 252 may be extended in the length direction of the spine to be a predetermined length in the second expansion zone 220, and the length of the first blocking member 252 may be set to be less than a length of the second expansion zone 220. Thus, the first blocking member 252, which is provided as a plurality of first blocking members, may be disposed in the second expansion zone 220 along the length direction of the spine.

In addition, the first blocking member 252, which is provided as a plurality of first blocking members, may be disposed to be separate from one another in a lateral direction against the length direction of the spine. Thus, a space may be formed between neighboring blocking members among the first blocking member 252.

Here, the flow path for air flow may be formed through the space between the blocking members disposed to be separate from one another in the length direction of the spine in the second expansion zone 220. Thus, air may be conveyed from an end of the second expansion zone 220 towards the first expansion zone 210, or conveyed from the first expansion zone 210 towards the second expansion zone 220.

As described above, the second expansion zone 220 may act as a path through which air is conveyed, and may also maintain an expanded state by retaining the air itself.

A plurality of air columns 224 may be formed in the space between the first blocking member 252 disposed to be separate from one another in the lateral direction against the length direction of the spine in the second expansion zone 220.

The plurality of air columns 224 may be disposed to be separate from one another, with the first blocking member 252 being therebetween, in the lateral direction against the length direction of the spine in the second expansion zone 220.

For example, as illustrated in FIGS. 4A through 4C, when air is not injected through the fluid inlet 222 or when the air is removed through the fluid inlet 222, the plurality of air columns 224 may be contracted, and thus an entire size of the compression belt 200 may decrease.

For another example, as illustrated in FIGS. 5A through 5C, when air is injected through the fluid inlet 222, a plurality of air columns 232 may be expanded, and thus the size of the compression belt 200 may increase in the length direction of the spine in comparison to the example of the plurality of air columns 224 illustrated in FIGS. 4A through 4C.

In addition, the third expansion zone 230 may include the second blocking member 254 corresponding to the first blocking member 252, with the first expansion zone 210 being disposed therebetween. The third expansion zone 230 may include the plurality of air columns 232 corresponding to the plurality of air columns 224 in the second expansion zone 220, with the first expansion zone 210 being disposed therebetween.

A detailed description of the second blocking member 254 and the plurality of air columns 232 in the third expansion zone 230 will be omitted here because the second blocking member 254 and the plurality of air columns 232 correspond to the first blocking member 252 and the plurality of air columns 224 in the second expansion zone 220.

The first blocking member 252 provided in the second expansion zone 220 and the second blocking member 254 provided in the third expansion zone 230 may facilitate bending of the compression belt 200, and thus it is obvious that the compression belt 200 may effectively cover the torso.

Air conveyance in the second expansion zone 220 and the third expansion zone 230 is described in the foregoing, and air conveyance in the first expansion zone 210 will be described hereinafter.

Dissimilarly to the second expansion zone 220 and the third expansion zone 230, the first expansion zone 210 may not include a blocking member.

However, an attachable and detachable portion 240 may be disposed at a center of the first expansion zone 210 to be attached to or detached from the back face of the third frame 130.

For example, the attachable and detachable portion 240 may be provided in a quadrangular shape, and may include an attachable and detachable member such as Velcro to correspond to the attachable and detachable member 132 in the third frame 130.

FIGS. 4A and 5A illustrate a surface facing the spine, and the attachable and detachable member may be provided in the attachable and detachable portion 240.

Here, inflow of air into an internal space of the attachable and detachable portion 240 may be blocked.

Thus, the air conveyed to the first expansion zone 210 from the second expansion zone 220 may make a detour by the attachable and detachable portion 240, and the air may be conveyed through an upper and lower space of the second expansion zone 220.

As described above, since the inflow of the air into the attachable and detachable portion 240 is blocked, the attachable and detachable portion 240 may be stably attached and detached between the compression belt 200 and the support frame 100 and the flow path for conveying a fluid into the second expansion zone 220 may be formed.

In addition, some of the first blocking member 252 and the second blocking member 254 may be disposed at both ends of the first expansion zone 210, and thus fluid conveyance may be enabled between the second expansion zone 220 and the first expansion zone 210 and fluid conveyance may be enabled also between the first expansion zone 210 and the third expansion zone 230.

Further, an attachable and detachable member 260 may be provided at both ends of the compression belt 200 to correspond to each other at the ends of the compression belt 200, in order to maintain a state in which the compression belt 200 is being worn on the user. Also, when the spine support and traction apparatus 10 is not used, the compression belt 200 may be readily detached from the torso of the user using the attachable and detachable member 260.

Referring back to FIG. 1A, when the compression belt 200 is contracted, a separation distance between the first blocking member 252 and the second blocking member 254 disposed separately from each other at the both ends of the first expansion zone 210 or a width of the first expansion zone 210 may correspond to a width of the third frame 130. In addition, when the compression belt 200 is contracted, a length of the first expansion zone 210 may be less than a separation distance between the first frame 110 and the second frame 120.

Referring back to FIG. 1B, when the compression belt 200 is expanded, the separation distance between the first blocking member 252 and the second blocking member 254 disposed separately from each other at the both ends of the first expansion zone 210 or the width of the first expansion zone 210 may correspond to the width of the third frame 130 or may be slightly greater than the width of the third fame 130. In addition, when the compression belt 200 is expanded, the length of the first expansion zone 210 may correspond to the separation distance between the first frame 110 and the second frame 120.

Further, when the compression belt 200 is expanded, an external force may be applied in all outer directions of the attachable and detachable potion 240 of the first expansion zone 210, and the external force may be transferred to the support frame 100 to act as a tractive force for the spine, and may thus widen a gap between spinal joints of the spine.

According to example embodiments described herein, a spine support and traction apparatus may generate a tractive force for an entire spine by a compression belt that applies an external force to a support frame configured to cover a pelvis/lumbar vertebra, and may allow the support frame to be more closely attached to the spine by the compression belt, and thus may prevent the support frame from being excessively separate from the spine.

According to example embodiments described herein, a spine support and traction apparatus may include a portion of a support frame configured to cover a pelvis/lumbar vertebra and another portion of the support frame configured to cover a back/thoracic vertebra to support a spine, and may effectively generate a tractive force for the spine by applying an external force to the support frame by expansion of a compression belt.

The compression belt may allow the support frame to be more closely attached to the spine, and thus may prevent the support frame from being excessively separate from the spine.

The compression belt may include a blocking member to form a flow path through which a fluid moves and facilitate bending of the compression belt.

The compression belt and the support frame may be attachable and detachable, and thus replacing the compression belt and the support frame may be readily performed. In addition, the support frame may be disassembled, and thus replacing and newly assembling the support frame to be suitable for a body type of a user may be readily performed.

The spine support and traction apparatus may be conveniently wearable for anyone irrespective of time and place, and may alleviate a muscular pain or help treat spinal diseases in real life.

A number of example embodiments have been described above. Nevertheless, it should be understood that various modifications may be made to these example embodiments. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. A spine support and traction apparatus, comprising:
a support frame extended in a length direction of a spine; and
a compression belt connected to the support frame and configured to apply an external force to the support frame, and
wherein the compression belt comprises an expansion zone, and
wherein the expansion zone is provided to generate traction force for the spine by expansion of the expansion zone.

2. The apparatus of claim 1, wherein the support frame comprises:
a first frame configured to cover a pelvis;
a second frame disposed separately from the first frame in the length direction of the spine and configured to cover a torso; and
a third frame extended in the length direction of the spine to connect the first frame and the second frame.

3. The apparatus of claim 2, wherein the expansion zone is provided as a plurality of expansion zones, and
wherein the plurality of expansion zones comprises:
a first expansion zone disposed in the third frame and configured to apply an external force to the third frame;
a second expansion zone connected to one end of the first expansion zone and configured to cover one side of the torso; and
a third expansion zone connected to another end of the first expansion zone and configured to cover another side of the torso, and
wherein the second expansion zone or the third expansion zone comprises a fluid inlet into which a fluid is injected to contract or expand the plurality of expansion zones.

4. The apparatus of claim 3, wherein the compression belt further comprises an attachable and detachable portion configured to attach and detach the compression belt to and from the third frame, and
wherein the attachable and detachable portion is disposed at a center of the first expansion zone.

5. The apparatus of claim 4, wherein inflow of the fluid into the attachable and detachable portion is blocked, and the fluid flowing in the first expansion zone makes a detour around the attachable and detachable portion.

6. The apparatus of claim 3, wherein the second expansion zone and the third expansion zone each comprise a plurality of blocking members to block inflow of the fluid, and the fluid is conveyed through a space between neighboring blocking members among the plurality of blocking members.

7. The apparatus of claim 6, wherein the plurality of blocking members is extended in the length direction of the spine in the second expansion zone and the third expansion zone, and is disposed separately from one another in a lateral direction against the length direction of the spine.

8. The apparatus of claim 6, wherein blocking members among the plurality of blocking members are disposed at both ends of the first expansion zone, and a separation distance between blocking members disposed at one end of the first expansion zone and blocking members disposed at the other end of the first expansion zone corresponds to a width of the third frame.

9. The apparatus of claim 3, wherein the first expansion zone is contracted or expanded by the fluid injected into the fluid inlet, and
wherein, when the first expansion zone is contracted, a length of the first expansion zone is less than a separation distance between the first frame and the second frame, and
when the first expansion zone is expanded, the length of the first expansion zone corresponds to the separation distance between the first frame and the second frame.
